# FASCICULE DE BREVET EUROPEEN

(11) **EP 0 580 621 B1**
(45) Date de publication et mention de la délivrance du brevet: **03.06.1998**
(21) Numéro de dépôt: 92907135.5
(22) Date de dépôt: 13.02.1992
(51) Int. Cl.: C12Q 1/68, C12N 15/12, C12N 15/70, C12N 15/79

(54) **FRAGMENT D'ACIDE NUCLEIQUE DE LA REGION DU CHROMOSOME X IMPLIQUE DANS LE SYNDROME X FRAGILE, SONDE NUCLEOTIDIQUE ET PROCEDE POUR LE DIAGNOSTIC DU RETARD MENTAL AVEC X FRAGILE**
NUKLEINSÄURE FRAGMENT DES X-CHROMOSOMEN BEREICHS BETEILIGT AM EMPFINDLICHEN X-SYNDROM, NUKLEOTIDISCHE SONDE UND VERFAHREN FÜR DIE DIAGNOSE VON GEISTIGER ZURÜCKGEBLIEBENHEIT MIT EMPFINDLICHEM X
NUCLEIC ACID FRAGMENT OF THE X CHROMOSOME REGION INVOLVED IN THE FRAGILE X SYNDROME, NUCLEOTIDE PROBE AND METHOD OF DIAGNOSING MENTAL RETARDATION WITH FRAGILE X

(30) Priorité: 13.02.1991 FR 9101684
(43) Date de publication de la demande: 02.02.1994
(73) Titulaire: INSTITUT NATIONAL DE LA SANTE ET DE LA RECHERCHE MEDICALE (INSERM), 75654 Paris Cédex 13 (FR)
(72) Inventeur: MANDEL, Jean-Louis, F-67300 Schiltigheim (FR); ROUSSEAU, François, Sainte Foy, Québec G1W 3H5 (CA); VINCENT, Anne, F-69160 Tassin la Demi Lune (FR); HEITZ, Dominique, F-67000 Saverne (FR); OBERLE, Isabelle, décédé (FR)
(74) Mandataire: Desaix, Anne
(86) Numéro de dépôt international: FR9200145
(87) Numéro de publication internationale: WO9214840

(56) Documents cités:
- WO-A-86/05512
- WO-A-90/05194
- WO-A-91/09140
- WO-A-92/12262
- AMERICAN JOURNAL OF HUMAN GENETICS, vol. 48, no. 1, janvier 1991, US; F. ROUSSEAU et al., pp. 108-116/
- AMERICAN JOURNAL OF HUMAN GENETICS, vol. 47, no. 2, août 1990, US; G. SUTHERS et al., pp. 187-195/
- GENOMICS, vol. 9, no. 1, 1991, US; G.K. SUTHERS et al., pp. 37-43/
- PROCEEDINSG OF THE NATL. ACADEMY OF SCIENCES USA, vol. 87, no. 10, mai 1990, Washington, DC (US); S.T. WARREN et al., pp. 3856-3860/
- SCIENCE, vol. 251, no. 4998, 08 mars 1991, pages 1236-1239, Washington, DC (US); D. HEITZ et al., pp. 1236-1239/
- CELL, vol. 64, no. 4, 22 février 1991, Cambridge, MA (US); M.V. BELL et al., pp. 861-866/
- GENOMICS, vol. 10, no. 1, 1991, US; M.C. HIRST et al., pp. 243-249/

## Description

Le syndrome de retard mental avec X fragile est une maladie liée au chromosome X qui atteint environ un garçon sur mille cinq cent. Les femmes vectrices, environ une sur sept cent cinquante, ont un risque de 30% de présenter une débilité légère ou même moyenne. Le syndrome du retard mental avec X fragile constitue la cause la plus fréquente de retard mental héréditaire.

Le diagnostic de la maladie est établi par une analyse cytogénétique délicate qui nécessite des conditions de culture particulières et l'analyse de 50 à 100 mitoses, car le site fragile détectable cytogénétiquement n'est présent que dans 5 à 50% des mitoses. Le diagnostic des femmes vectrices est encore plus difficle car seules 50 à 60% de ces femmes expriment le site fragile, dans une proportion généralement faible des mitoses.

La difficulté du test cytogénétique fait qu'il n'est pas pratiqué de manière fiable que dans un nombre limité de laboratoires ; en outre, le diagnostic de la maladie n'est souvent pratiqué qu'après la naissance d'un deuxième enfant atteint dans la même famille ; or l'établissement précoce d'un tel diagnostic est capital pour mettre en oeuvre des investigations permettant le diagnostic prénatal et le dépistage des femmes vectrices qui transmettent la maladie à 50% de leurs enfants mâles, dans les familles à risque. La haute fréquence de la maladie et l'importance génétique précoce justifient un dépistage systématique néonatal de la maladie.

La présente invention vise précisément à fournir de nouveaux moyens d'investigation et de diagnostic, basés sur l'étude directe de l'ADN, permettant un diagnostic rapide de la maladie et applicables à un nombre important de patients.

Le syndrome du retard mental avec X fragile se caractérise par la présente d'un site fragile dans la région Xq27.3, induit in vitro par une privation en acide folique ou par des inhibiteurs de la synthèse des déoxynucléotides. La cartographie génétique de la région Xq27-q28 a permis de confirmer que le locus de la maladie est localisé au niveau ou à côté du site fragile (Oberle et al, Proc. Natl. Acad. Sci., 83, 1016-1020, 1986 ; Suthers et al, Science, 248, 1298-1300, 1989 ; Rousseau et al, J. Hum. Genet., 48, 108-116, 1991).

Il apparaît par ailleurs que la transmission héréditaire de la maladie est inhabituelle par rapport aux autres maladies liées au chromosome X. On a constaté que des hommes phénotypiquement normaux transmettent la mutation à leurs filles, lesquelles présentent rarement les symptômes de la maladie, par contre les fils et filles de ces femmes présentent un fort taux d'apparation de la maladie (Sherman et al, Hum. Genet., 69, 3289-3299, 1985). Plusieurs hypothèses ont été proposées pour expliquer ce phénomène.

L'invention fournit également des moyens pour la détection, chez des personnes non malades, d'anomalies et notamment de mutations au niveau du site X fragile.

Ces personnes non malades sont dites porteuses ou vectrices.

Les marqueurs génétiques proposés dans l'art antérieur ne peuvent servir qu'à suivre la transmission de la mutation dans les familles où la maladie a déjà été diagnostiquée par une étude cytogénétique, il s'agit d'un diagnostic indirect par étude de liaison ; il est en général nécessaire d'utiliser plusieurs marqueurs de part et d'autre du locus de la maladie, et ces marqueurs ne sont informatifs que dans des proportions variables de familles.

La demande de brevet internationale WO90/05194 décrit un nouveau locus intitulé 1A1 distant du site X fragile mais dont le polymorphisme de restriction permet de disposer d'un marqueur génotypique. Les sondes capables de détecter le locus 1A1 permettent le diagnostic de plusieurs maladies liées au chromosome X et notamment le syndrome du X fragile, par hybridation sélective au niveau de la région Xq27. - DXS52 dudit chromosome X.

D'autres sondes proches du locus X fragile ont également été décrites, notamment par les inventeurs. Ainsi F. Rousseau et al (Am. J. Hum. Genet. (1991) 48:108-116) rapportent la mise en évidence de sept régions proches du locus X fragile (FRAXA). Deux de ces régions comprises entre DXS369 et DXS296 contiennent les marqueurs génétiques les plus proches de FRAXA connus à ce jour, l'une de ces régions contient deux sondes détectant un polymorphisme. Ces sondes dénommées St677 (DX463) et 2-34 (DXS477) détectent toutes deux les fragments de restriction Taq1 polymorphes ; une autre sonde détectant également un polymorphisme, et dénommée Do33 (DXS465 : 42% hétérozygocité) est localisée dans la même région que DXS296, proximale au point de clivage du chromosome correspondant au locus du syndrome Hunter (IDS). L'utilisation de ces sondes a permis de déterminer la disposition physique des régions précédentes : Cen-DXS369-DXS476-(DXS463,DXS477) -(DXS296,DXS465)-IDS-DXS304-tel, et leur position par rapport au locus X fragile.

L'analyse par électrophorèse en champ pulsé du locus X fragile avec les sondes précédentes et plus particulièrement avec la sonde Do33 (DXS465) a conduit les inventeurs à remarquer que les patients avec X fragile et retard mental se distinguent des sujets normaux ou des sujets porteurs de la mutation X fragile mais sans expression clinique de la maladie, par une inhibition totale ou partielle de la digestion de sites de restriction proches de la sonde Do33, et notamment de sites pour les enzymes BssHII, SacII, EagI.

Les inventeurs ont clone sous forme de chromosome artificiel de levure (YAC), le segment d'ADN d'environ 225 kb défini entre les sondes St677 et Do33, et situé dans la région Xq27-q28 du chromosome X humain. Des sondes provenant de ce segment ont été préparées pour l'analyse des mutations, des méthylations de l'ADN et des gènes impliqués dans le syndrome X fragile.

Ces sondes ont notamment permis l'étude, à partir d'ADN de leucocytes, de la digestibilité des sites de restriction BssHII, EagI, SacII et de manière générale des sites de restriction dont la digestion par l'enzyme correspondant est inhibée lorsque le site contient une cytosine méthylée ; ces sites sont dénommés sites à CpG, il s'agit par exemple de sites contenant le dinucléotide CpG, tels que HpaII, CCGG ou HhaI, GCGC, ou de sites susceptibles de recouvrir partiellement un dinucléotide CpG tels que AvaII GG(A ou T)CC, dans une séquence CGG(A ou T)CC ou GG(A ou T)CGG ou encore BanI (GG Py Pu CC) ; les inventeurs ont mis en évidence une corrélation surprenante entre l'expression clinique de la maladie et l'inhibition de la digestibilité des sites à CpG dans la région d'environ 20 kb autour de la position 125 kb définie sur le segment d'ADN cloné par les inventeurs, à environ 100 kb de la sonde Do33 en direction du centromère.

Ces sondes sont également appropriées pour la détection du locus X fragile, sans manifestation clinique de la maladie, qui résulte de la mutation de l'acide nucléique de la région du chromosome X décrite ci-dessus, par allongement de l'ADN.

L'allongement peut en effet être plus ou moins important selon que le sujet est seulement porteur de l'anomalie ou qu'il présente des signes cliniques de retard mental associé à l'anomalie X fragile. Les sondes de l'invention permettent avantageusement la détection de prémutations correspondant à un allongement de l'acide nucléique d'environ 100pb (paires de bases) à environ 500pb et la détection de mutations complètes correspondant à un allongement de l'acide nucléique d'au moins environ 500pb, pouvant aller jusqu'à plus de 3000pb. Les prémutations correspondent en général à un sujet porteur de l'anomalie sans manifestation clinique, alors que les mutations par allongement de taille supérieure à 500pb sont normalement associées avec la présence de signes cliniques.

Dans la suite le terme mutation sera utilisé pour désigner les deux situations évoquées ci-dessus et la distinction entre les mutations associées ou non à des signes cliniques sera faite si nécessaire.

Des sondes intéressantes dans le cadre de la recherche de mutation de la région X fragile sans expression clinique du retard mental, sont par exemple les sondes dites adjacentes au site à CpG.

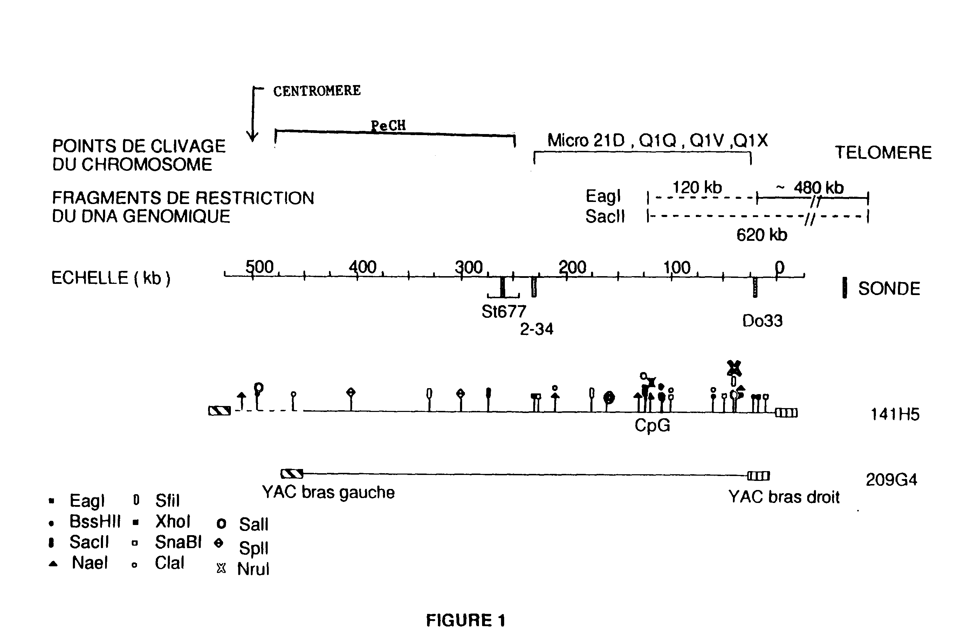

La figure 1 est une représentation schématique de la région du chromosome X autour de la position 125 kb se situant à environ 100 kb de ka sonde Do33 en direction du centromère. Sur l'échelle, on a représenté la localisation des sondes St677, 2-34, et Do33. En concordance avec l'échelle on a représenté les clones YAC 141 H5 et 209G4 correspondant aux segments d'ADN sus-mentionnés, et la localisation de sites de restriction EagI, BssII etc..., a été représentée sur la ligne du clone 141 H5. L'indication CpG sur la ligne du clone 141 H5 correspond à la région dont la méthylation est anormale chez les patients atteints de retard mental avec X fragile.

Ce phénomène d'inhibition de digestibilité des sites CpG peut être détecté par la méthode de Southern Blot, en utilisant des sondes provenant de la région de 20 kb autour de la position 125 kb définie à la figure 1, sondes proches ou contenant les sites à CpG.

Les inventeurs ont également mis en évidence par la même méthode, des anomalies de migration de fragments d'ADN, non nécessairement liées au phénomène de méthylation, anomalies qui sont caractéristiques des sujets porteurs d'une mutation X fragile.

Ces anomalies ont été détectées notamment dans l'ADN digéré par les enzymes EcoRI ou HindIII après hybridation avec une sonde spécifique de la susdite région.

Il apparaît que les mutations germinales responsables du syndrome X fragile et susceptibles d'être transmises à la descendance se situent dans la région du chromosome X définie entre les sondes St677 et Do33, et la méthylation des sites à CpG dans cette région est une conséquence non obligatoire de ces mutations, conséquence qui est liée à l'expression clinique de la maladie.

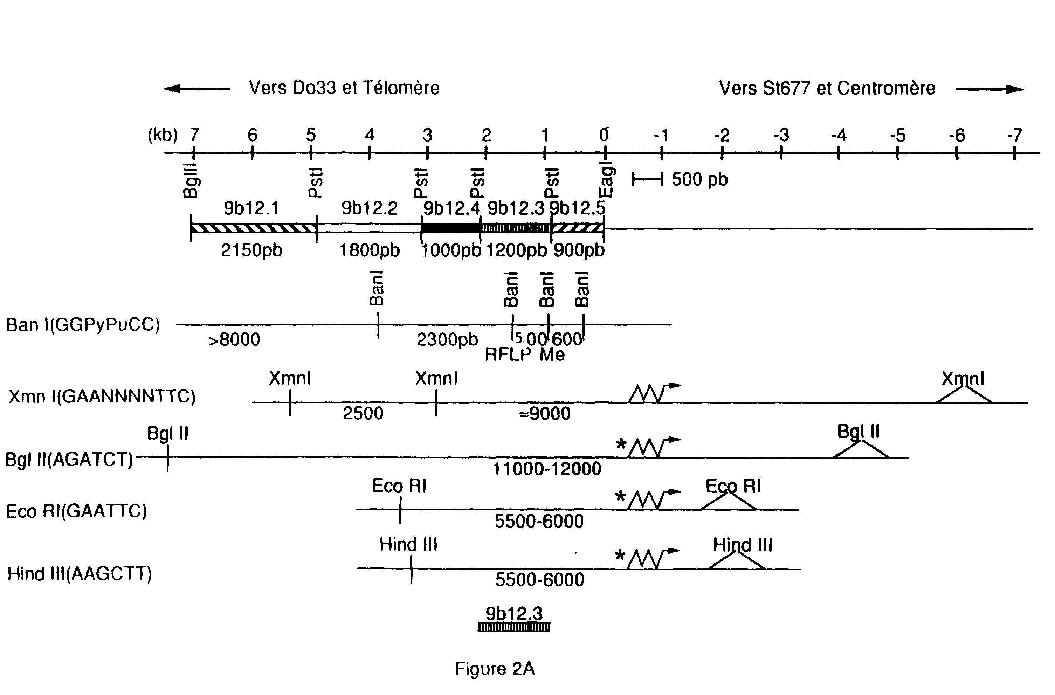

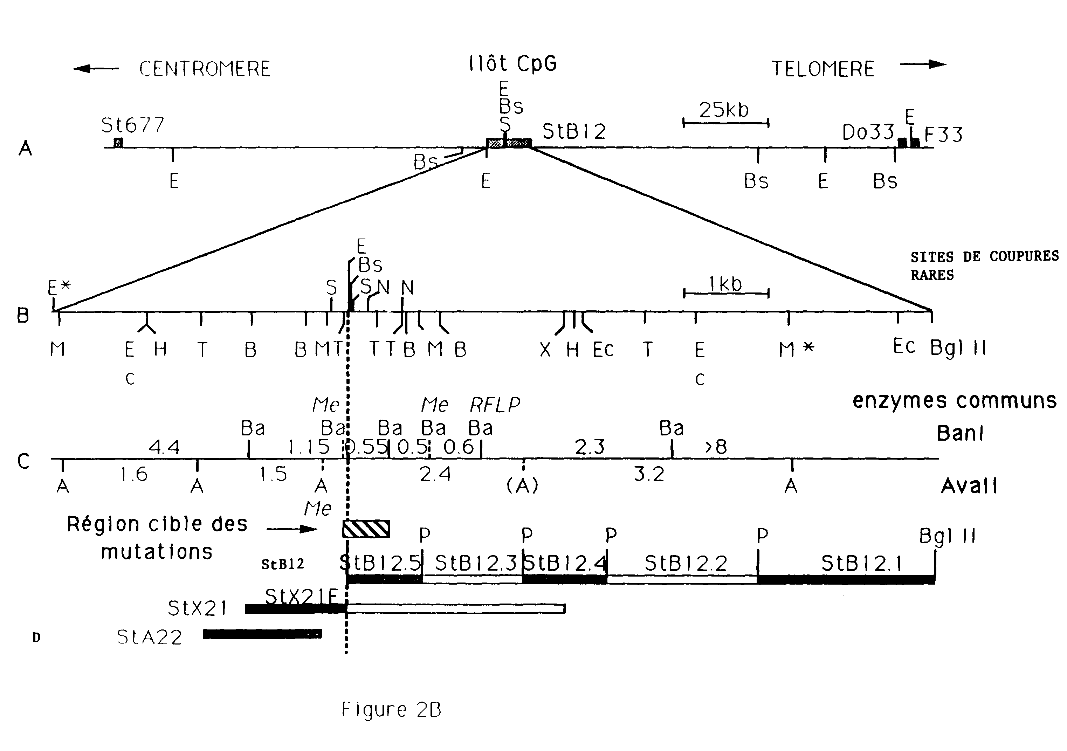

Les anomalies de migration de fragments d'acide nucléique, corrélées à un site X fragile, chez les sujets porteurs (vecteurs) sans expression clinique nécessairement associée, résultent notamment d'un allongement du fragment d'ADN génomique autour du site X fragile. Cet allongement peut par exemple être la conséquence de l'insertion d'une séquence additionnelle. Cette allongement peut dans certains cas résulter de l'amplification (répétition) d'une courte séquence contenue au voisinage du site X fragile, par exemple au niveau du fragment 9B12.5 près du site EagI, représenté à la figure 2.

L'invention vise en conséquence tout fragment d'acide nucléique issu de la région Xq27-q28 du chromosome X, adjacent ou contenant les sites à CpG, situé dans la région de 10 à 20 kb autour de la position 125 kb.

De manière générale, l'invention concerne tout fragment d'acide nucléique compris dans le segment d'ADN d'environ 225 kb, défini entre les sondes St677 et Do33. De préférence de tels fragments d'acide nucléique comprennent au moins 15pb ou nucléotides.

Les séquences d'acide nucléique de l'invention sont des séquences d'ADN, d'ADNc ou encore d'ARNm.

Les séquences d'acide nucléique de l'invention sont des séquences individualisées et par conséquent elles ne sont pas dans leur environnement naturel.

L'ADNc correspondant aux séquences d'acide nucléique de l'invention peut être obtenu selon les techniques conventionnelles par criblage d'une banque d'ADNc humain disponible, avec un fragment d'acide nucléique de l'invention, le criblage permettant de détecter les fragments d'ADNc qui hybrident sélectivement avec le fragment d'acide nucléique de l'invention.

Cette région du chromosome X est impliquée dans le syndrome X fragile et les sondes spécifiques de cette région sont avantageusement utilisées pour détecter la présence d'anomalies de méthylation liées à l'expression clinique de la maladie, ou pour détecter la présence de mutations responsables du syndrome X fragile et de sa transmission, ou encore pour localiser, identifier et analyser l'expression du ou des gènes impliqués dans le syndrome X fragile. Ces sondes peuvent en général être utilisées pour détecter les anomalies de l'ADN qui sont la conséquence directe ou indirecte des mutations associées au syndrome X fragile.

L'invention concerne donc toute sonde nucléotidique capable de s'hybrider sélectivement avec un fragment d'acide nucléique compris dans le segment d'ADN défini entre les sondes St677 et Do33 et plus particulièrement avec un fragment d'acide nucléique adjacent ou contenant les sites à CpG dans la région d'environ 20 kb autour de la position 125 kb.

Les sondes de l'invention s'hybrident spécifiquement dans des conditions de stringence appropriées avec les fragments d'acide nucléique susmentionnés mais ne s'hybrident pas avec d'autres régions du génome humain dans des mêmes conditions d'hybridation.

Une telle hybridation comporte les étapes suivantes :
- le traitement de pré-hybridation d'un support, comme un filtre de nitrocellulose ou une membrane de nylon, sur lequel est fixé l'ADN à tester susceptible de s'hybrider avec les sondes susmentionnées, à une température de 42°C et pendant 2 heures avec une solution de pré-hybridation ayant la composition suivante : 40% de formamide ; 0,9 M NaCl ; 200 µg/ml d'ADN de sperme de saumon ; 50 mM de sodium phosphate pH 6,5 ; 4% de dextran sulfate ; 0,08% (poids/volume) de ficoll et de polyvinyl pyrrolidone,
- l'hybridation dans une solution identique à la solution de pré-hybridation, comprenant en outre au moins une sonde nucléotidique conforme à l'invention avantageusement marquée, à une température de 42°C et pendant 18 heures,
- 4 lavages successifs,
- la révélation de l'hybride éventuellement formé par toute méthode adaptée au marquage de la sonde.

Les conditions d'hybridation définies ci-dessus constituent des conditions préférées, mais ne sont nullement limitatives et peuvent être modifiées sans affecter les propriétés d'appariement des sondes de l'invention et les séquences de nucléotides susmentionnées.

Les sondes de l'invention sont avantageusement marquées par tout marqueur classiquement utilisé. Elles peuvent être marquées à l'aide d'un traceur radioactif comme ³²P, ³⁵S, ¹²⁵I, ³H, ¹⁴C et le marquage radioactif peut être réalisé selon une méthode quelconque connue de l'Homme du Métier.

Les sondes peuvent être marquées en 3' par addition d'un ou plusieurs désoxynucléotides ou ribonucléotides ou d'un didéoxynucléotide, marqué en alpha par le ³²P, en présence de la Terminal Déoxynucléotidyl Transférase, ou en 5' par transfert d'un groupe phosphate radioactif d'un déoxynucléotide ou didéoxynucléotide libre marqué en position gamma, en présence de la T4 DNA ligase. Les sondes peuvent encore être marquées en utilisant un ADN polymérase par "Nick Translation" ou "Random Priming" ou "Polymerase Chain Reaction".

La méthode de détection de l'hybridation dépendra du marqueur radioactif utilisé et pourra reposer sur l'autoradiographie, la scintillation liquide, le comptage gamma ou toute autre autre technique permettant de détecter l'émission du rayonnement émis par le marqueur radioactif.

Un marquage non radioactif peut également être utilisé, en associant aux sondes des groupements présentant des propriétés immunologiques comme un antigène, une affinité spécifique pour certains réactifs comme un ligand, des propriétés physiques comme la fluorescence ou la luminescence, des propriétés permettant la complétion des réactions enzymatiques comme une enzyme ou un substrat d'enzyme. Le marquage non radioactif peut être fait également directement par modification chimique de l'ADN, comme la photobiotinylation ou la sulfonation.

Les sondes de l'invention sont utilisées pour la détection in vitro, des mutations responsables du syndrome X fragile, ainsi que des anomalies de méthylation des sites à CpG ou d'autres anomalies de l'ADN conséquentes à la présence de ces mutations et associées au syndrome.

La détection de mutation vise à suivre la transmission familiale et permet notamment un diagnostic des personnes non atteintes susceptibles de transmettre la maladie, telles que des femmes vectrices ou des hommes normaux transmetteurs de la mutation.

La détection des anomalies de méthylation permet un diagnostic de la maladie, exprimée cliniquement ou susceptible de l'être dans le cadre d'un diagnostic anténatal ou à la naissance.

D'une manière générale, les procédés de détection des mutations responsables du syndrome X fragile, ainsi que des anomalies de méthylation des sites à CpG, ou d'autres anomalies de l'ADN associées au syndrome, reposent sur l'utilisation d'au moins une sonde conforme à l'invention et d'au moins une enzyme de restriction appropriée de manière à ce que le fragment détecté par la sonde soit différent selon qu'il contienne ou non la mutation ou l'anomalie de méthylation ou toute autre anomalie de l'ADN.

Un tel procédé peut être réalisé selon la méthode dite "Southern Blot" (Sambrook, Fritsh and Maniatis ; Molecular cloning : A laboratory manual (1989), Cold Spring Harbor Laboratory Press), comprenant les étapes suivantes :
- traitement de l'ADN génomique extrait par exemple de leucocytes ou de villosités choriales ou de toute autre source d'ADN génomique, par au moins une enzyme de restriction déterminée, dans des conditions permettant l'obtention de fragments de restriction issus du clivage de l'ADN au niveau de ses sites de restriction reconnus par ladite enzyme ;
- séparation des fragments par électrophorèse ;
- mise en contact d'une sonde nucléique selon l'invention susceptible de s'hybrider spécifiquement avec les fragments précédents dans des conditions permettant la formation de complexes d'hybridation ;
- détection des complexes d'hybridation ;
- mesure de la taille des fragments de restriction engagés dans les complexes d'hybridation.

La recherche d'anomalies de méthylation pour le diagnostic in vitro du syndrome X fragile chez des patients atteints de retard mental est avantageusement réalisée selon le procédé précédent mettant en oeuvre au moins une enzyme de restriction à site sensible à la méthylation, avec au moins une sonde conforme à l'invention avantageusement marquée.

Le procédé sus-mentionné peut être mis en oeuvre avec une seule enzyme à site de restriction sensible à la méthylation telle que BssHII, EagI, AvaII, BanI ; ou encore avec deux enzymes, l'une à site de restriction sensible à la méthylation et l'autre à site de restriction non sensible à la méthylation. Les sondes de l'invention utilisées pour l'hybridation avec les fragments obtenus sont avantageusement adjacentes ou contiennent le site pour l'enzyme à site sensible à la méthylation.

La méthode de Southern Blot est également utilisée pour détecter les mutations associées au syndrome X fragile, ou les mutations existant chez les personnes vectrices, sans signes cliniques. Ces mutations sont par exemple des délétions ou des insertions de séquence d'ADN, ou d'autres anomalies de l'ADN telles que des anomalies de conformation ou de réplication, qui sont conséquentes à ces mutations.

La détection de ces mutations pour le diagnostic in vitro du syndrome X fragile ou plus généralement de la mutation du site X fragile, chez des patients atteints ou non de retard mental est réalisée selon la méthode de Southern Blot en soumettant l'ADN génomique à une digestion par toute enzyme de restriction appropriée non nécessairement sensible à la méthylation telle que EcoRI ou HindIII. Les fragments de restriction sont mis en évidence par hybridation avec une sonde de l'invention avantageusement marquée et la migration électrophorétique anormale desdits fragments est corrélée soit avec l'expression clinique de la maladie, soit avec la présence de la mutation même en l'absence même d'expression clinique de la maladie.

Cette migration anormale des fragments correspond à la présence de fragments allongés par la mutation insertionnelle d'une séquence de nucléotides.

L'invention concerne aussi les kits pour la mise en oeuvre du procédé précédent.

Parmi les fragments d'acide nucléique de l'invention, ceux de 20 à 30 nucléotides peuvent être avantageusement utilisés en tant qu'amorces dans un procédé d'amplification génétique d'un fragment d'acide nucléique de la région de 20kb autour de la position 125. L'amplification peut être destinée à révéler la présence de mutations au niveau de cette région, notamment par allongement de l'ADN, ou/et detinée à la détection d'un fragment contenant les sites à CpG. Ce procédé d'amplification consiste à fixer spécifiquement par hybridation sur l'ADN génomique extrait par exemple de leucocytes, deux oligonucléotides judicieusement choisis parmi ceux contenant les fragments d'acide nucléique de 20 à 30 nucléotides de l'invention qui sont adjacents au fragment à amplifier, puis à mettre en oeuvre un processus d'extension enzymatique à l'aide d'ADN- polymérase, suivi d'un processus de dénaturation, et à répéter le cycle hybridation-extension-dénaturation connu sous le nom de cycle PCR "Polymerase Chain Reaction", un nombre de fois suffisant pour augmenter la quantité du fragment à amplifier dans une proportion exponentielle par rapport au nombre de cycles effectués.

La méthode d'amplification décrite peut être appliquée à la détection d'anomalies de l'ADN qui est la conséquence de la présence des susdites mutations.

Le diagnostic in vitro du syndrome X fragile, notamment chez des patients atteints de retard mental, ou chez des personnes vectrices, est avantageusement réalisé par une amplification génétique effectuée en parallèle sur l'ADN digéré par au moins une enzyme de restriction choisie parmi les enzymes non sensibles à la méthylation ou au contraire sensibles à la méthylation, et sur l'ADN non digéré. L'analyse à des fins de diagnostic est faite en comparant le produit d'amplification de l'ADN non digéré à celui de l'ADN digéré par l'enzyme ou les enzymes de restriction choisies. Cette analyse des produits d'amplification peut être réalisée par visualisation de la fluorescence induite par le bromure d'ethidium, après électrophorèse sur gel d'agarose ou de polyacrylamide, ou par la détection du marquage radioactif ou non radioactif, tel que par un système biotine-avidine, des oligonucléotides amorces ou des nucléotides incorporés dans le fragment amplifié. Le nombre de cycles d'amplification est limité de façon à conserver une linéarité de l'amplification et donc une estimation quantitative de la digestibilité des sites de l'enzyme ou des enzymes utilisées. Avantageusement des marqueurs internes sont utilisés, notamment d'autres fragments du génome co-amplifié contenant également un site de l'enzyme choisie, en tant que témoin de l'action de l'enzyme de restriction, et en tant que témoin de l'efficacité de l'amplification, un fragment ne contenant pas un tel site.

La mesure quantitative des fragments caractéristiques des mutations, en particulier résultant de l'allongement, ainsi que la mesure quantitative de la méthylation de l'ADN génomique au site étudié peut se faire par adaptation de la méthode d'amplification décrite précédemment, notamment en utilisant en plus des oligonucléotides selon l'invention, des oligonucléotides dits "linkers" et de l'ADN Ligase, méthode dénommée "Ligation mediated PCR" décrite par Steigerwald et al (Nucleic ACids Res. (1980) 18:1435-1439).

Les délétions ou tout autre réarrangement constituant des mutations susceptibles de donner lieu au syndrome X fragile en particulier par allongement d'acide nucléique, peuvent être également détectés par la méthode d'amplification d'ADN dite multiplex, à l'aide de couples d'oligonucléotides pouvant être mélangés dans une seule réaction pour amplifier plusieurs fragments de la région autour de la position 125 kb et détecter la disparition ou la modification de certains de ces fragments. La détection de mutations ponctuelles est avantageusement réalisée par amplification des segments d'ADN, suivie d'une analyse par les méthodes de SSCP (single strand conformation polymorphism) ou de DGGE (denaturing gradient gel electrophoresis), ou encore de détection d'hétéroduplex.

L'invention concerne également les kits pour la mise en oeuvre du procédé précédent. De tels kits comprennent :
- des oligonucléotides marqués ou non pour l'amplification de fragments d'acide nucléique présentant une anomalie résultant d'une mutation, notamment par allongement de l'ADN et/ou de fragments d'acide nucléique contenant un site sensible à la méthylation selon l'invention ;
- des oligonucléotides marqués ou non pour l'amplification des segments d'acide nucléique témoin ;
- des oligonucléotides dits "linkers" ;
- des tampons, nucléotides et enzymes propices à l'amplification.

Un groupe de sondes particulièrement avantageuses pour la réalisation de l'invention, comprend les sondes dont la description détaillée est donnée dans la partie expérimentale. Ces sondes comprennent les fragments 9B12.3 (désigné maintenant StB12.3), 9B12.5 (StB12.5), 9B12.4, 9B12.2 et 9B12.1 localisés par rapport à l'ADN situé entre les sondes Do33 et St677, représenté à la figure 2. D'autres sondes sont les fragments stA22 (AvaII-AvaII) d'environ 1500bp, stX21 d'environ 3800bp et stX21E (centromérique du site EagI).

Avantageusement la sonde 9B12.3 peut être mise en oeuvre pour détecter des anomalies au niveau du site X fragile de fragments d'acide nucléique, et plus spécialement des mutations par allongement de l'ADN, après une étape de digestion avec l'enzyme de restriction EcoRI.

Des résultats analogues sont obtenus en utilisant l'enzyme de restriction HindIII.

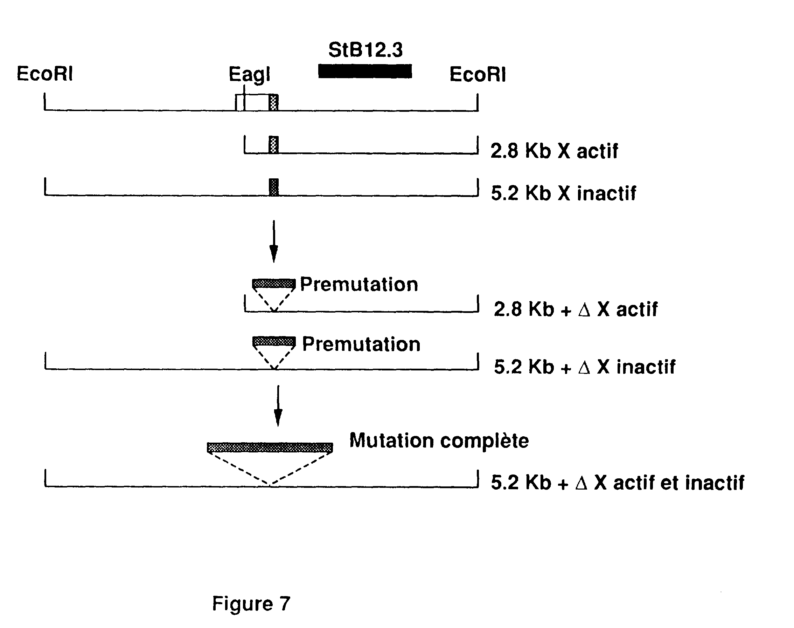

Un schéma des résultats obtenus sur des fragments de restriction résultant de la digestion par EcoRI, détectés par la sonde 9B12.3, montrant un fragment normal ou muté est donné à la figure 7.

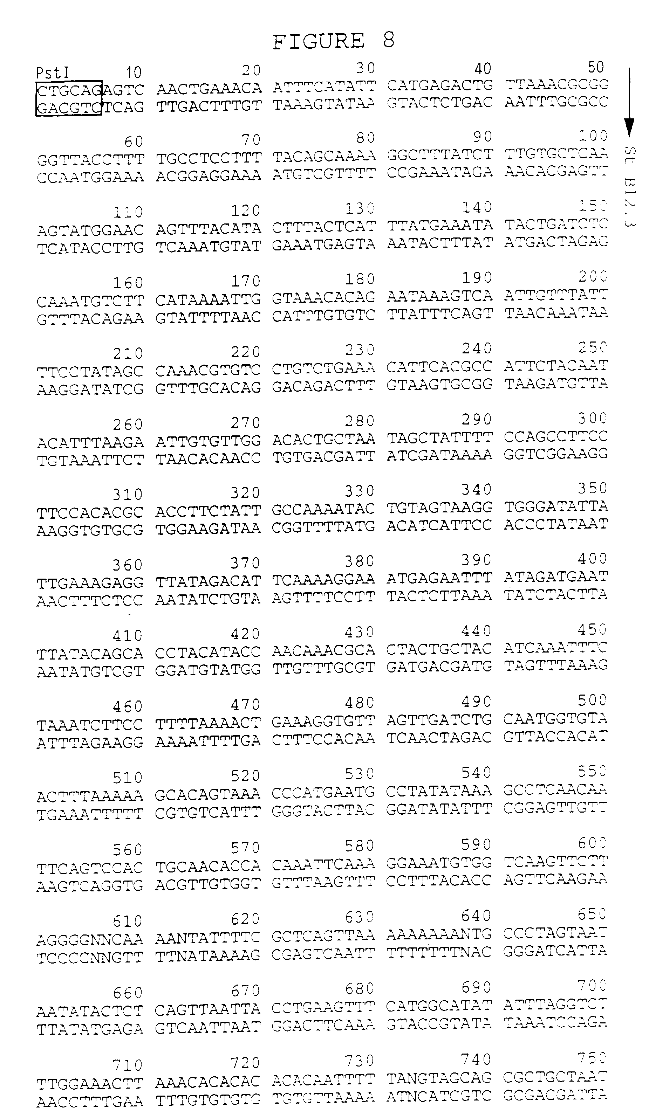

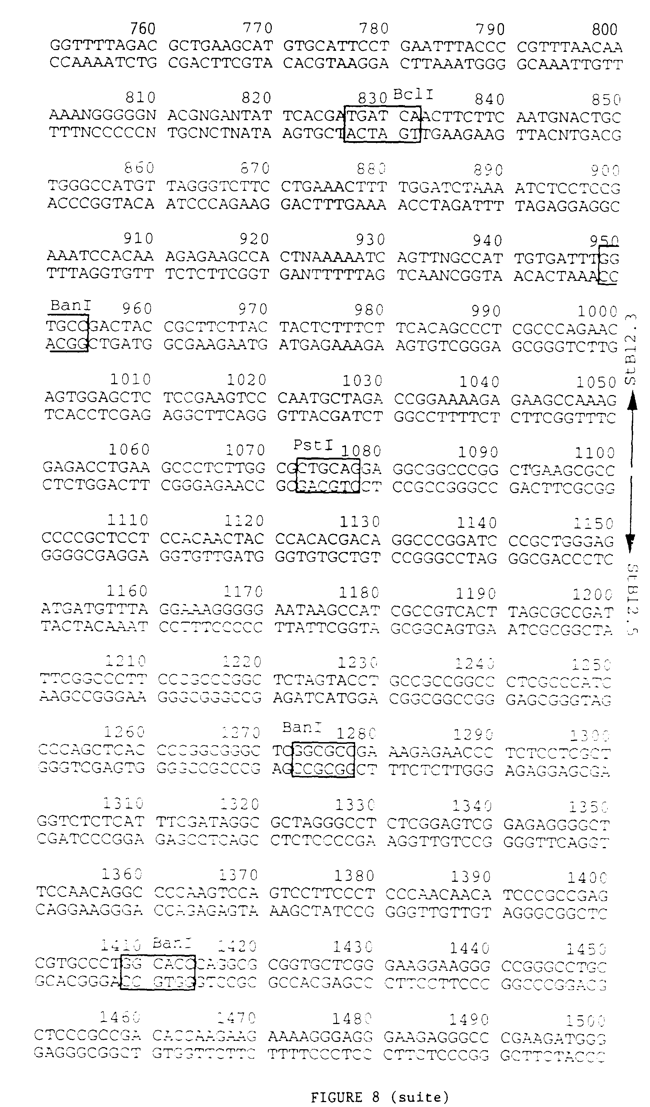

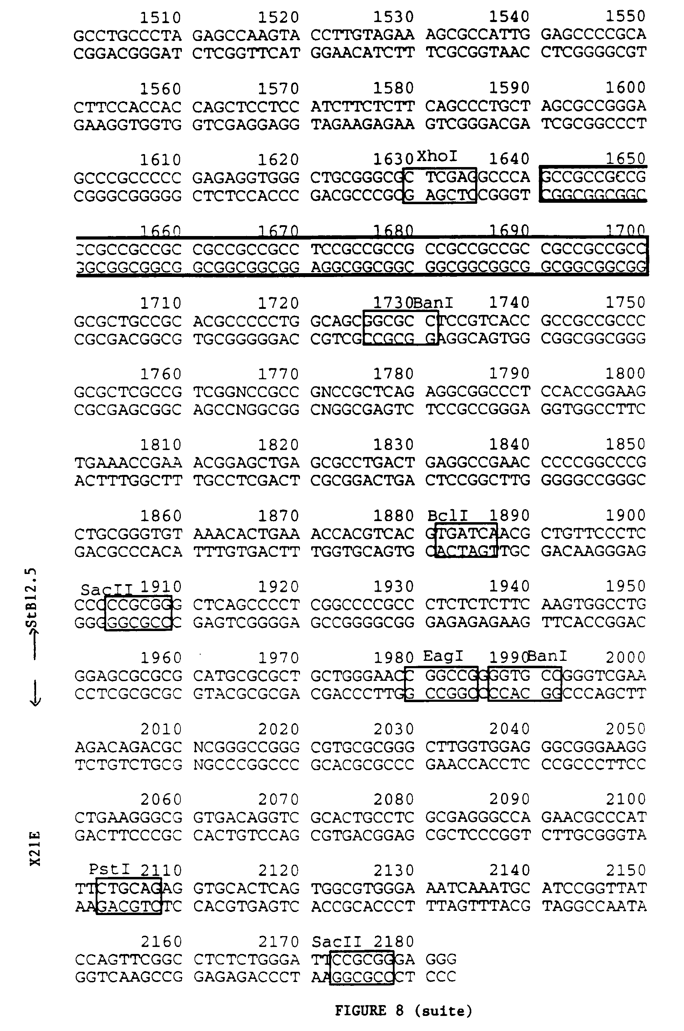

La séquence nucléotidique des sondes 9B12.3 (stB12.3) et 9B12.5 (stB12.5) est représentée à la figure 8, avec sa séquence complémentaire.

En faisant par ailleurs appel aux sondes décrites ci-dessus, pour la révélation des fragments d'ADN auxquels on a préalablement fait subir une double digestion par une enzyme sensible à la méthylation (par exemple EagI) et par une enzyme insensible à la méthylation (par exemple EcoRI) on peut révéler la présence de la mutation au niveau du site X fragile et l'état de méthylation.

Les sondes de l'invention permettent également l'accès à la région dans laquelle se situent le ou les gènes impliqués dans l'expression clinique du syndrome X fragile. En effet, l'identification de sites CpG tels que BssHII, EagI, SacII dans la région proche de la position 125 kb, indique la présence de gènes adjacents, dont l'expression est contrôlée par cette région (îlot CpG). Les sondes correspondant à ces gènes sont utiles pour la détection des ARN messagers correspondants, ou pour la construction de vecteurs d'expression des protéines correspondantes en vue d'application thérapeutique ou pour la production d'anticorps spécifiques susceptibles également d'être utilisés en diagnostic.

L'Homme de l'Art dispose des moyens permettant d'identifier voire de sélectionner ceux des fragments d'acide nucléique correspondant aux gènes présents dans la région entre les sondes St677 et Do33 de part et d'autre de la position 125 kb, lesquels gènes sont susceptibles d'être inactivés directement par mutation ou indirectement en raison de la méthylation des sites CpG présents autour de la position 125 kb.

A cet égard un fragment selon l'invention est caractérisé en ce qu'il hybride sélectivement avec un ARNm présent dans des cellules humaines, par exemple des leucocytes et en ce qu'il correspond à un gène dont l'extrémité 5' est contenue ou adjacente à l'ilôt CpG en position 125 kb sur la carte génétique représentée à la figure 2A.

Un autre fragment particulier de l'invention est caractérisé en ce qu'il est compris dans l'ADN défini entre la sonde Do33 et la position 125 kb à environ 100 kb de la sonde Do33 de la région Xq27-q28 du chromosome X, et ce qu'il hybride sélectivement avec un ARNm présent dans des cellules humaines, par exemple des leucocytes.

A ce titre, l'invention concerne tout ADN recombinant contenant un fragment d'acide nucléique de l'invention associé à toute séquence permettant la transcription et/ou la traduction dans la cellule hôte dans laquelle ledit ADN recombinant est susceptible d'être introduit. L'invention concerne aussi en conséquence les vecteurs de clonage et d'expression contenant un des fragments d'acide nucléique de l'invention.

L'introduction de l'ADN recombinant est avantageusement réalisée à l'aide d'un vecteur, notamment du type plasmidique, apte à se répliquer dans la cellule hôte et à y permettre la transcription de l'ARN correspondant et l'expression des protéines codées par le fragment d'acide nucléique contenu dans l'ADN recombinant.

Les cellules hôtes sus-mentionnées peuvent être des cellules procaryotes, notamment des cellules de E.coli, ou, d'une manière plus avantageuse, des cellules eucaryotes, qui permettent d'obtenir des protéines glycosylées et matures (levures, cellules CHO, ou cellules d'insectes infectées par le bacculovirus). Ces protéines peuvent avantageusement être utilisées pour la préparation d'anticorps spécifiques utiles pour le diagnostic du syndrome X fragile et dans des applications thérapeutiques.

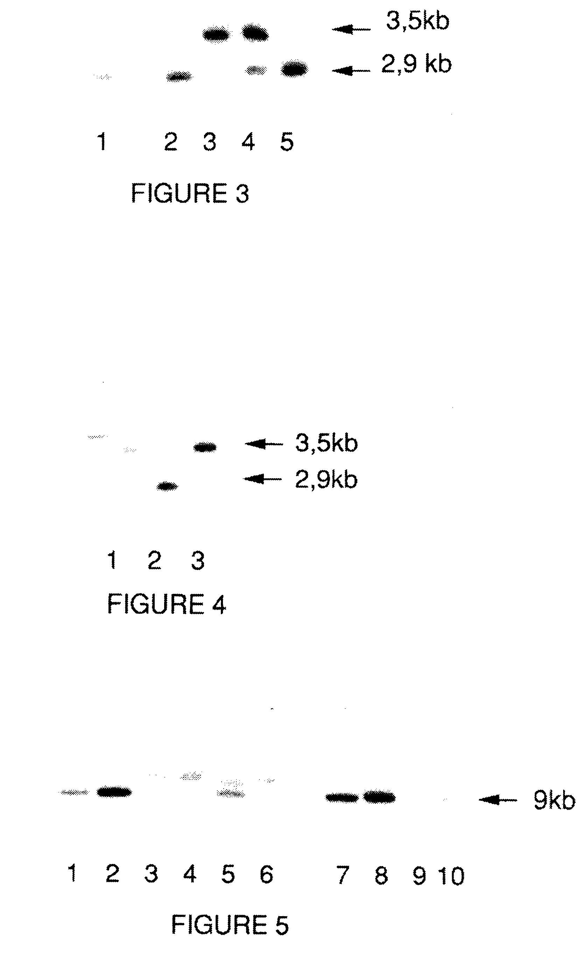

Outre les caractéristiques qui précèdent, l'invention comporte d'autres caractéristiques qui apparaîtront au cours de la description qui suit et qui se réfèrent à des exemples de mise en oeuvre de la présente invention et qui sont illustrés par la figure, étant entendu que ces exemples ne sauraient constituer une quelconque limitation à la portée de l'invention.
- Figure 1: représentation schématique de la région du chromosome X autour de la position 125 kb. Les sondes St677, 2-34 et Do33 sont localisées. Les segments d'ADN des clones YAC 141 H5 et 20964 sont localisés.
   L'indication CpG sur la ligne du clone 141 H5 correspond à la région dont la méthylation est anormale chez les patients atteints de retard mental avec X fragile.
- Figure 2: représentation schématique de la carte de la sonde 9B12 et de ses sous-fragments. L'orientation est inversée par rapport à celle de la figure 1.
   Figure 2A :
   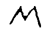 bandes floues (chez les patients ou les personnes susceptibles de transmettre la maladie)
   * tailles anormales
- Figure 3: représentation schématique d'une image d'autoradiographie sur gel d'agarose à 0,9%, d'ADN de leucocytes ou de villosités choriales d'homme normaux (1 et 5), d'un homme avec X fragile (3), d'une femme vectrice (4) et d'une femme normale, digérés par BanI et révélés par la sonde 9B12.3.
- Figure 4: représentation schématique d'une image d'autoradiographie sur gel d'agarose à 0,9%, d'ADN de villosités choriales d'un homme normal (2), d'un homme avec X fragile (3), d'un foetus avec X fragile, digérés par BanI et révélés par la sonde 9B12.3.
- Figure 5: représentation schématique d'une image d'autoradiographie sur gel d'agarose à 0,9%, d'ADN de leucocytes d'hommes normaux (1 et 10), d'hommes avec retard mental lié au X fragile (3, 4, 6, 9), de femmes vectrices (5, 7, 8) et d'une femme normale (2), digérés par XmnI et révélés par la sonde 9B12.3.

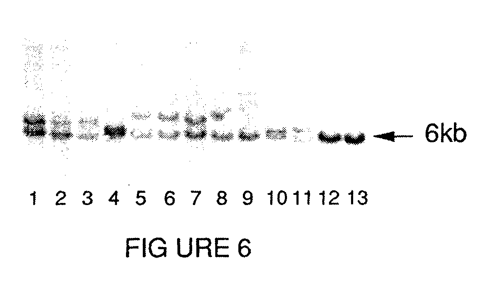

- Figure 6: représentation schématique d'une image d'autoradiographie sur gel d'agarose à 0,9%, d'ADN de leucocytes de femmes normales (9, 12, 13) et de femmes vectrices (1 à 8, 10 et 11) de la mutation X fragile n'exprimant pas toutes des signes clinique ou cytogénétique, digérés par EcoRI et révélés par la sonde 9B12.3.
- Figure 7: résultats de la détection des anomalies au niveau du site X fragile de fragments d'acide nucléique digérés par EcoRI et détectés par la sonde 9B12.3.
- Figure 8: séquence nucléotidique des sondes 9B12.3 (stB12.3) et 9B12.5 (stB12.5) ainsi que la séquence complémentaire.

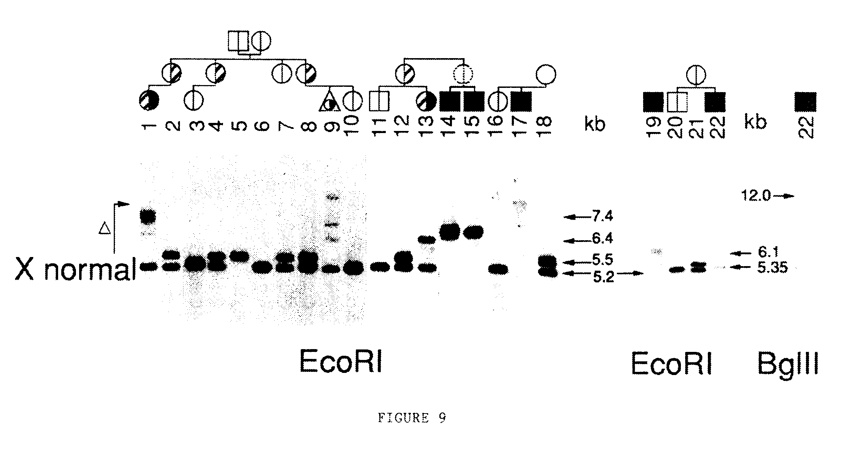

- Figure 9: représentation de la détection de mutations au niveau de la région X fragile, par digestion de l'ADN avec EcoRI au moyen de la sonde stB12.3.

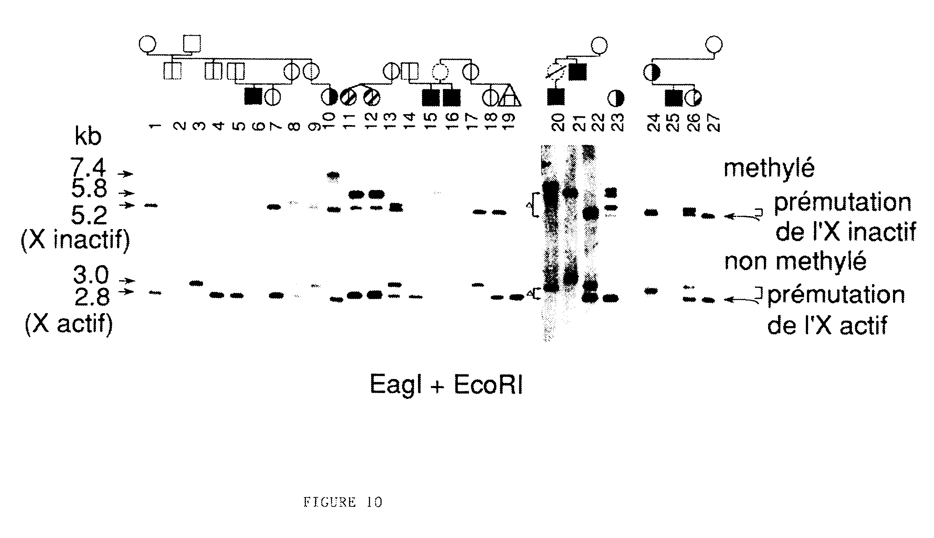

- Figure 10: analyse des résultats de détection de familles présentant le syndrome X fragile, après double digestion de l'ADN avec EcoRI et EagI. La détection est faite avec la sonde stB12.3.

### I - Préparation des sondes St677 et Do33

Des segments d'ADN correspondant aux sondes St677 et Do33 peuvent être obtenus à partir d'ADN humain par amplification génétique en utilisant les oligonucléotides suivants :
- pour ST 677 5'ACTCCTTCACAAATCTTCCG3' et 5'ATCATGCAAGACAGTTCTCTGC3'
- pour Do33 5'GTGAAGCAATTTGAGTTGGTGGGAG3' et

5'CCCAAGTTTAGCTTCCGTAGAAGCC3'

Les conditions d'amplification dans un milieu standard sont les suivantes :
- hybridation : 1 minute à 60°C,
- élongation : 8 minutes à 72°C,
- dénaturation : 1 minute à 94°C.

### II - Préparation des sondes de l'invention

Les sondes de l'invention ont été obtenues par sous-clonage des fragments d'acide nucléique de l'invention provenant de la digestion de segments d'ADN contenus dans la région d'environ 225 kb définie entre les sondes St677 et Do33, et localisés dans la région Xq27-q28 du chromosome X humain, cloné sous forme de chromosome artificiel de levure (YAC) et représenté à la figure 1.

En particulier le sous-clonage vise à obtenir des sondes spécifiques de fragments situés dans la région de 20 kb autour de la position 125 kb et correspondant donc spécifiquement aux fragments d'acide nucléique adjacents ou comprenant les sites à CpG. A titre d'exemple une telle sonde est obtenue par sous-clonage de fragments préparés par double digestion EagI, BglI du clone 141H5. La sonde obtenue 9B12 correspond à un fragment EagI-BglI d'environ 7 kb contenant le site EagI à la position 125 kb sur la carte de la figure 1.

La figure 2 est une représentation schématique de la carte de la sonde 9B12 et de ses sous-fragments 9B12.1, 9B12.2, 9B12.3, 9B12.4 et 9B12.5, orientés de façon inverse de la figure 1. Cette figure comporte 7 lignes suivantes :
- la ligne (1) est une échelle en kb sur laquelle est mentionnée en 0 la position 125 kb définie sur la carte du segment cloné de la figure 1 entre les sondes St677 et Do33 à environ 100 à 125 kb de la sonde Do33.
- la ligne (2) est une représentation en concordance avec l'échelle de la sonde 9B12 et de ses sous-fragments 9B12.1, 9B12.2, 9B12.3, 9B12.4 et 9B12.5.
- la ligne (3) représente la localisation, en concordance avec l'échelle, des sites BanI (GGPyPuCC) et notamment le site BanI polymorphique noté RFLP et le site BanI sensible à la méthylation noté Me.
- la ligne (4) représente la localisation, en concordance avec l'échelle, des sites XmnI (GAANNNNTTC).
- la ligne (5) représente la localisation, en concordance avec l'échelle de la ligne (1), des sites BglII (AGATCT).
- la ligne (6) représente la localisation, en concordance avec l'échelle de la ligne (1), des sites EcoRI (GAATCC).
- la ligne (7) représente la localisation, en concordance avec l'échelle de la ligne (1), des sites HindIII (AAGCT).

Les cartes des sites BanI, XmnI et EcoRI correspondent aux fragments altérés chez les porteurs de mutation X fragile.

### III- Diagnostic du retard mental X fragile par détection d'anomalies de méthylation selon la méthode de Southern Blot

L'ADN génomique extrait de leucocytes d'un patient atteint de retard mental, est coupé par les enzymes AvaII, BanI ou XmnI. Les fragments d'ADN obtenus sont analysés par électrophorèse sur gel d'agarose 0,9% en tampon TAE, puis transférés sur une membrane de nylon.

La membrane sur laquelle sont fixés les fragments d'ADN est soumise à un traitement de pré-hybridation à 42°C pendant 2 heures avec une solution ayant la composition suivante : 40% formamide ; 0,9 M NaCl ; 200 µg/ml d'ADN de sperme de saumon ; 50 mM de sodium phosphate pH 6,5 ; 4% de dextran sulfate ; 0,8% (poids/volume) de ficoll et de polyvinyl pyrrolidone ; puis la solution de pré-hybridation est remplacée par une solution d'hybridation identique à la solution de pré-hybridation, et comprenant en outre la sonde 9B12.3 marquée au dCTP alpha ³²P et d'activité spécifique 2 10⁸ dpm/µg ; après incubation pendant 18 heures à 42°C et lavages successifs avec une solution 0,5 SSC à 65°C, on effectue une autoradiographie à -80°C.

Tout ou partie des fragments AvaII, BanI ou XmnI détectés chez les sujets normaux est remplacée chez les sujets exprimant le syndrome X fragile par des fragments plus grands. En particulier avec l'enzyme XmnI, l'apparition de plusieurs bandes assez proches les unes des autres est très caractéristique des sujets ayant une expression phénotypique du syndrome.

Cette distinction entre anomalies liées à l'expression clinique et/ou cytogénétique et anomalies présentes chez des personnes porteuses de mutation X fragile sans expression clinique et/ou cytogénétique est explicitée dans les exemples suivants de détection desdites anomalies par la méthode de Southern Blot.

### Anomalies liées à l'expression clinique et/ou cytogénétique

### a - Digestion par l'enzyme BanI (site GG Py Pu CC)

La figure 3 est une représentation schématique d'une image d'autoradiographie sur gel d'agarose à 0,9%, d'ADN de leucocytes ou de villosités choriales d'hommes normaux (1 et 5), d'un homme avec X fragile (3), d'une femme vectrice (4) et d'une femme normale, digérés par BanI et révélés par la sonde 9B12.3.

La figure 4 est une représentation schématique d'une image d'autoradiographie sur gel d'agarose à 0,9%, d'ADN de villosités choriales d'un homme normal (2), d'un homme avec X fragile (3), d'un foetus avec X fragile, digérés par BanI et révélés par la sonde 9B12.3.

La sonde 9B12.3 détecte dans l'ADN génomique d'un homme normal, après digestion par l'enzyme BanI, soit un seul fragment de 2,9 kb (allèle 1) ou deux fragments de 2,3 et 0,6 kb (allèle 2). L'existence de deux allèles est due à la présence d'une site BanI polymorphique indiqué RFLP sur la figure 2. Chez un patient mâle atteint de retard mental avec X fragile porteur de l'allèle 1, le fragment de 2,9 kb est remplacé plus ou moins complètement par un fragment de 3,5 kb. Chez un patient porteur de l'allèle 2, le fragment de 0,6 kb est remplacé plus ou moins complètement par un fragment de 1,2 kb. Ces différences spécifiques, chez le mâle, du retard mental avec X fragile sont dues à la méthylation d'un site BanI, indiqué Me sur la figure 2. Ce site est inclu dans la séquence suivante : TCGGTGCCGA, compatible avec une inhibition par méthylation de la cytosine.

L'anomalie a été détectée non seulement sur ADN de leucocytes, mais également sur ADN de villosités choriales de foetus porteurs de mutation X fragile. Elle est donc utilisable en diagnostic post-natal ou anténatal chez les mâles.

Les fragments de 3,5 kb et 1,2 kb peuvent par contre être détectés chez les femmes normales (méthylation du site BanI sur le chromosome X inactif). Toutefois, leur intensité apparaît plus forte chez les femmes vectrices avec une expression clinique ou cytogénétique du syndrome X fragile.

### b - Digestion avec l'autre enzyme XmnI (site GAANNNNTTC)

La figure 5 est une représentation schématique d'une image d'autoradiographie sur gel d'agarose à 0,9%, d'ADN de leucocytes d'hommes normaux (1 et 10), d'hommes avec retard mental lié au X fragile (3, 4, 6, 9), de femmes vectrices (5, 7, 8) et d'une femme normale (2), digérés par XmnI et révélés par la sonde 9B12.3

La sonde 9B12.3 détecte un fragment de 9 kb chez les hommes et les femmes ainsi que chez les porteurs de mutation X fragile n'exprimant pas cliniquement ou cytogénétiquement la maladie. Chez les hommes atteints de retard mental avec X fragile, le fragment de 9 kb est remplacé en tout ou partie par des fragments de taille apparente supérieure à 10 kb. L'apparition de tels fragments est également caractéristique des femmes vectrices avec expression clinique et/ou cytogénétique.

### IV- Diagnostic d'anomalies présentes chez les personnes porteuses de mutation X fragile sans expression clinique et/ou cytogénétiqne

La figure 6 est une représentation schématique d'une image d'autoradiographie sur gel d'agarose à 0,9%, d'ADN de leucocytes de femmes normales (9, 12, 13) et de femmes vectrices (1 à 8, 10 et 11) de la mutation X fragile n'exprimant pas toutes des signes clinique ou cytogénétique, digérés par EcoRI et révélés par la sonde 9B12.3

La sonde 9B12.3 détecte un fragment EcoRI d'environ 6 kb chez un homme normal ou une femme normale. Par contre chez une femme vectrice de mutation X fragile, un fragment additionnel plus grand, de taille variable selon les individus (6 à 7 kb) est présent. Cette anomalie apparaît chez les femmes vectrices sans expression clinique et/ou cytogénétique, et peut donc être utilisée pour le diagnostic des femmes vectrices. Chez les hommes atteints, le fragment normal est remplacé par un fragment ou une série de fragments de taille apparente plus élevée. Des résultats très semblables sont obtenus avec l'enzyme HindIII.

La figure 9 est une autre représentation de la détection de mutations au niveau de la région X fragile, par digestion de l'ADN avec EcoRI au moyen de la sonde stB12.3.

Figure 9 : détection des mutations X fragile dans les produits de digestion d'ADN génomique par EcoRI. L'ADN de leucocytes a été totalement digéré par EcoRI. Les flèches indiquent la taille du fragment normal (5,2 kb) et certains fragments mutés. Sur la gauche Δ fait référence à l'accroissement de taille du fragment muté. Les symboles décrivant les sujets et leurs relations à l'intérieur de la famile sont présentés en haut. Les carrés et les ronds représentent respectivement les hommes et les femmes. Les symboles ouverts qui ne sont pas coupés en deux indiquent les sujets qui sont apparemment normaux et qui n'ont pas été testés cytogénétiquement. Pour les symboles coupés en deux, la partie droite représente la séquence du site X fragile et la partie gauche le degré de retard mental. Les symboles coupés en deux, en blanc coïncident avec des sujets normaux avec aucun syndrome X fragile, les symboles hachurés correspondent à des sujets avec un retard mental faible et une expression X fragile dans moins de 4% des cellules et les symboles pleins correspondent à des sujets avec retard mental et expression X fragile dans plus de 4% des cellules. Les symboles contenus dans un triangle correspondent à des échantillons obtenus d'un foetus. Les symboles en pointillés montrent des sujets pour lesquels la digestion par EcoRI a été faite et qui n'apportent pas de renseignement pour clarifier la relation familiale des autres sujets. L'échantillon à ligne 22 avait un nuage faible à 7,5 kb dans le produit de digestion par EcoRI. Ce nuage est plus visible comme une bande à 3 kb dans le produit de digestion par BglII du même échantillon, montré sur la droite ; il est localisé juste au dessus du fragment BglII normal qui a une longueur de 12 kb.

### V - Diagnostic du retard mental avec X fragile par amplification génétique du fragment contenant une anomalie de méthylation

L'ADN génomique extrait de leucocytes d'un patient atteint de retard mental, est soumis à une digesiton par une enzyme à site sensible à la méthylation telle que AvaII, BanI ou XmnI et l'amplification est réalisée en parallèle sur l'ADN génomique digéré par l'enzyme de restriction sensible à la méthylation, et sur l'ADN non digéré.

Deux oligonucléotides de 20 à 30 nucléotides adjacents au fragment d'ADN génomique à amplifier contenant le site pour l'enzyme sensible à la méthylation, sont hybridés avec l'ADN soumis à la digestion et l'ADN non soumis à la digestion. Puis un processus d'extension enzymatique à l'aide d'ADN-polymérase, suivi d'un processus de dénaturation est mis en oeuvre ; le cycle hybridation-extension-dénaturation est répété un nombre de fois suffisant pour augmenter la quantité du fragment contenant les sites à CpG dans une proportion exponentielle par rapport au nombre de cycles effectués.

Le nombre de cycles d'amplification est limité de façon à conserver une linéarité de l'amplification et donc une estimation quantitative de la digestibilité du site de l'enzyme sensible à la méthylation. Avantageusement des marqueurs internes sont utilisés, notamment d'autres fragments du génome co-amplifié contenant un site de l'enzyme sensible à la méthylation, non méthylé chez l'homme, ou de fragments ne contenant pas un tel site.

L'analyse des produits d'amplification est réalisée par visualisation de la fluorescence induite par le bromure d'éthidium, après électrophorèse sur gel d'agarose ou de polyacrylamide. Si le site sensible à la méthylation est méthylé, le fragment amplifié est présent dans les deux réactions, si le site sensible à la méthylation est non méthylé, le fragment amplifié est présent seulement dans la réaction avec l'ADN non digéré.

### VI- Détection de mutations du site X fragile et schéma de méthylation de l'ADN digéré par EcoRI et EagI

Des familles présentant le syndrome X fragile ont été analysées par double digestion de l'ADN avec EcoRI et EagI, un procédé qui a permis de révéler à la fois la présence de la mutation et l'état de la méthylation (EagI est incapable de couper l'ADN si le site de restriction riche en CpG est méthylé). Les résultats sont présentés à la figure 10. Pour cette double digestion, stB12.3 a hybridé avec l'ADN de toutes les personnes normales, avec un fragment de 2,8 kb EcoRI-EagI, correspondant au chromosome X actif (figure 10, lignes 2, 4, 5 et 14) ; chez les femmes il y a également eu hybridation avec un fragment EcoRI supplémentaire de 5,2 kb, correspondant au chromosome X inactif méthylé (lignes 1, 7, 18 et 27). Le fragment de 2,8 kb était absent chez les hommes présentant une mutation X fragile, alors que les femmes porteuses de la mutation avaient à la fois les fragments normaux (2,8 et 5,2 kb) et les fragments mutés. Les prémutations ont été détectées sous forme de fragments EcoRI-EagI de 2,9 à environ 3,3 kb chez les hommes (ligne 3) et ont été détectées sur le chromosome X actif des femmes (lignes 8, 24, 26 et semblables) alors que des fragments EcoRI de 5,3 à 5,7 kb correspondaient à une prémutation sur le chromosome X inactif. Les fragments correspondant à des mutations complètes n'ont pas été digérés par EagI et apparaissent donc comme des bandes simples ou hétérogènes au dessus de 5,7 kb (lignes 10, 12, 15 et semblables). Les cas de mosaïque (bande supplémentaire correspondant à une prémutation et présente en proportion variable) sont apparus sous forme d'un mélange de mutation complète (méthylée) et de prémutation non méthylée (lignes 20 et 21). Ceci est vérifié pour la plupart des cas de méthylation incomplète déjà reconnus chez les hommes avec le syndrome X fragile. Quelques personnes présentaient une bande partiellement non méthylée, plutôt homogène avec un accroissement de taille entre 600 à 1000bp. Ce schéma a été décrit comme "mosaïque de méthylation".

La figure 10 représente la détection des mutations X fragile et du schéma de méthylation pour l'ADN digéré par EcoRI et EagI : l'ADN de leucocytes a été digéré simultanément complètement avec les enzymes EcoRI et EagI. Les tailles sont indiquées sur la gauche pour certains des fragments mutés et pour les fragments normaux sur les chromosomes X actifs et inactifs (2,8 et 5,2 kb rspectivement). Δ correspond à l'accroissement de taille des fragments mutés. Les schémas d'hybridation sont identifiés sur la droite. Le trait "/" dénote un membre de la famille décédé. S'agissant des autres symboles se reporter à la figure 9. Les résultats pour les lignes 6 et 16 sont faibles. L'hybridation de ces échantillons à une sonde contrôle a montré que ces lignes contenaient autant d'ADN que les autres et ne présentaient pas d'artefact de migration.

## Revendications

1. Fragment d'acide nucléique localisé dans la région Xq27- q28 du chromosome X, caractérisé en ce qu'il est compris dans le segment d'ADN d'environ 225 kb défini entre les sondes St677 (ou DXS463) et Do33 (ou DXS465), et en ce qu'il contient au moins 15pb ou nucléotides.

2. Fragment d'acide nucléique selon la revendication 1, caractérisé en ce qu'il est adjacent aux sites à CpG ou en ce qu'il contient les sites à CpG, (EagI, BssHII, SacII), situé dans la région de 20 kb autour de la position 125 kb se situant à environ 100 kb de la sonde Do33 en direction du centromère.

3. Fragment d'acide nucléique, caractérisé en ce qu'il hybride sélectivement avec un fragment d'acide nucléique selon l'une quelconque des revendications 1 ou 2, et en ce qu'il s'agit d'ADN ou d'ARN.

4. Fragment d'acide nucléique selon la revendication 1 ou la revendication 2, caractérisé en ce qu'il hybride sélectivement avec un ARNm présent dans des cellules humaines, par exemple des leucocytes et en ce qu'il correspond à un gène dont l'extrémité 5' est contenue ou adjacente à l'ilôt CpG en position 125 kb se situant à environ 100 kb de la sonde Do33 en direction du centromère.

5. Fragment d'acide nucléique, caractérisé en ce qu'il s'agit de l'ADNc correspondant à l'ADN selon la revendication 4.

6. Fragment d'acide nucléique selon la revendication 1 ou la revendication 2, caractérisé en ce qu'il répond à l'une des séquences représentées à la figure 8.

7. Sonde nucléotidique pour détecter in vitro des mutations responsables du syndrome X fragile, ou d'autres anomalies conséquentes à la présence desdites mutations ou pour localiser le ou les gènes impliqués dans le syndrome X fragile, caractérisée en ce qu'elle correspond à un fragment d'acide nucléique selon l'une quelconque des revendications 1 à 6 ou en ce qu'elle est capable d'hybrider sélectivement avec un tel fragment.

8. Sonde nucléotidique, caractérisée en ce qu'elle est choisie parmi :
- les fragments 9B12 (EagI-BglI) d'environ 7000bp, 9B12.3 (PstI-PstI) d'environ 1200bp (PstI-PstI) d'environ 1000bp, 9B12.5 (EagI-PstI) d'environ 900bp, 9B12.2 (PstI-PstI) d'environ 1800bp, stA22 (AvaII-AvaII) d'environ 1500bp, stX21 d'environ 3800bp et stX21E (centromérique du site EagI) représentés à la figure 2B ;
- les fragments hybridant sélectivement avec l'un des fragments ci-dessus.

9. Sonde nucléotidique, caractérisée en ce qu'elle correspond au fragment 9B12.3 compris entre les nucléotides 1 et 1078 de l'enchaînement représenté à la figure 8, ou toute partie de ce fragment hybridant sélectivement avec un fragment d'acide nucléique selon l'une quelconque des revendications 1 à 6.

10. Sonde nucléotidique, caractérisée en ce qu'elle correspond au fragment 9B12.5 compris entre les nucléotides 1079 et 1985 de l'enchaînement représenté à la figure 8, ou toute partie de ce fragment hybridant sélectivement avec un fragment d'acide nucléique selon l'une quelconque des revendications 1 à 4.

11. Sonde nucléotidique selon l'une quelconque des revendications 8 à 10, caractérisée en ce qu'elle est marquée radioactivement, ou par un marqueur non radioactif, notamment un marqueur enzymatique, un ligand, un marqueur luminescent ou fluorescent.

12. ARN messager, notamment issu de leucocytes humains, caractérisé en ce qu'il hybride avec une sonde selon l'une quelconque des revendications 7 à 11.

13. Fragment d'acide nucléique, caractérisé en ce qu'il hybride sélectivement avec un ARN messager selon la revendication 12.

14. Procédé de détection in vitro d'anomalies de l'ADN conséquentes à la présence de mutations associées au syndrome X fragile ou existant chez des personnes ne présentant pas de signes cliniques ou d'autres anomalies de l'ADN conséquentes à la présence de ces mutations, caractérisé en ce qu'il comporte les étapes suivantes :
- le traitement de l'ADN génomique par au moins une enzyme de restriction à site sensible à la méthylation ;
- la séparation des fragments par électrophorèse et leur hybridation avec au moins une sonde selon l'une quelconque des revendications 8 à 11 ;
- la visualisation des hybrides formés et comparaison avec les résultats obtenus dans les mêmes conditions sur des fragments de contrôle.

15. Procédé de détection in vitro selon la revendication 14, caractérisé en ce que le traitement de l'échantillon d'ADN génomique comprend en outre l'utilisation d'une enzyme de restriction à site insensible à la méthylation et en ce que l'étape de visualisation des hybrides formés et de comparaison conduit à la détection de fragments de longueur supérieure à la longueur des fragments de contrôle obtenus dans les mêmes conditions, à partir d'un échantillon biologique prélevé chez un sujet normal, non porteur de mutation au site X fragile.

16. Procédé pour le diagnostic in vitro du syndrome X fragile ou de la présence de mutations chez des sujets porteurs sans manifestations cliniques de retard mental, selon la revendication 15, caractérisé en ce que la digestion de l'ADN génomique est réalisée par l'enzyme de restriction EagI à site sensible à la méthylation et une enzyme de restriction à site non sensible à la méthylation choisie parmi EcoRI ou HindIII.

17. Procédé de détection in vitro des mutations, associées au syndrome X fragile ou existant chez des personnes ne présentant pas de signes cliniques ou d'autres anomalies de l'ADN conséquentes à la présence de ces mutations, caractérisé en ce qu'il comporte les étapes suivantes :
- le traitement d'un échantillon d'ADN génomique avec au moins une enzyme de restriction à site insensible telle que EcoRI ou HindIII à la méthylation ;
- la séparation des fragments par électrophorèse et leur hybridation avec au moins une sonde selon l'une quelconque des revendications 8 à 11 ;
- la visualisation des hybrides formés et la détection de fragments de longueur supérieure à la longueur des fragments de contrôle obtenus par traitement dans les mêmes conditions, à partir d'un échantillon biologique prélevé chez un sujet normal, non porteur de mutation au site X fragile.

18. Procédé d'amplification génétique d'un fragment d'acide nucléique contenant des mutations ou d'autres anomalies, au sujet du site X fragile, le cas échéant conséquentes à ces mutations, caractérisé en ce qu'il consiste à fixer par hybridation sur l'ADN génomique deux oligonucléotides marqués flanquant ledit fragment, choisis parmi les fragments d'acide nucléique de 20 à 30 nucléotides hybridant sélectivement avec les fragments d'acide nucléique selon la revendication 1 ou 2, puis à mettre en oeuvre un processus d'extension enzymatique à l'aide d'ADN-polymérase, suivi d'un processus de dénaturation, et à répéter le cycle hybridation-extension-dénaturation un nombre de fois suffisant pour augmenter la quantité du fragment dans une proportion exponentielle par rapport au nombre de cycles effectués, dans des conditions permettant la visualisation des fragments amplifiés.

19. Procédé de diagnostic in vitro du syndrome X fragile, caractérisé en ce que l'on effectue en parallèle un procédé d'amplification génétique selon la revendication 18, d'une part sur de l'ADN soumis à la digestion par une enzyme à site sensible à la méthylation et d'autre part sur de l'ADN non soumis à la digestion par ladite enzyme, puis en ce que l'on compare les fragments obtenus visualisés au bromure d'éthidium ou détectés grâce au marquage des oligonucléotides amorces.

20. Procédé de détection de mutation associée au syndrome X fragile caractérisé en ce que l'on effectue une amplification génétique selon la revendication 18 ou 19 d'un ou plusieurs fragments d'acide nucléique de la région de 20 kb autour de la position 125 kb en particulier de la région contenant les sites proches de l'ilôt CpG, et en ce que l'on détecte la disparition ou la modification de certains de ces fragments, en particulier en ce que l'on détecte des fragments allongés par rapport à la taille des fragments obtenus dans les mêmes conditions de traitement, à partir d'un échantillon normal, non porteur de la mutation X fragile.

21. ADN recombinant contenant au moins un fragment d'acide nucléique selon l'une quelconque des revendications 1 à 6, le cas échéant associé à une séquence permettant la transcription et/ou la traduction dans une cellule hôte choisie dans laquelle ledit ADN recombinant est susceptible d'être introduit.

22. Vecteur de clonage et d'expression caractérisé en ce qu'il contient un ADN recombinant selon la revendication 21.

23. Hôte cellulaire caractérisé en ce qu'il est transformé par un vecteur recombinant selon la revendication 22.

24. Protéine résultant de la transcription et de la traduction d'un fragment d'ADNc selon la revendication 5 ou 6, telle qu'obtenue par les étapes de :
- transformation d'un hôte cellulaire déterminé par cet ADNc, dans des conditions permettant sa transcription en ARNm et sa traduction ;
- récupération de la protéine exprimée le cas échéant après lyse de l'hôte cellulaire.

25. Anticorps dirigés contre une protéine selon la revendication 24.

26. Kit pour le diagnostic in vitro du syndrome X fragile ou de la présence de mutations chez des sujets porteurs sans manifestations cliniques de retard mental, comprenant :
- des oligonucléotides répondant à une séquence d'un fragment d'acide nucléique selon l'une quelconque des revendications 1 à 6, marqués ou non pour l'amplification de fragments d'acide nucléique présentant une anomalie résultant d'une mutation, notamment par allongement de l'ADN et/ou de fragments d'acide nucléique contenant un site sensible à la méthylation selon l'invention ;
- des oligonucléotides marqués ou non pour l'amplification des segments d'acide nucléique témoin ;
- des oligonucléotides dits "linkers" ;
- des tampons, nucléotides et enzymes propices à l'amplification.

## Claims

1. Nucleic acid fragment situated in the Xq27-q28 region of the X chromosome, characterized in that it is comprised in the DNA segment of about 225 kb defined between the probes St677 (or DXS463) and Do33 (or DX5465), and in that it contains at least 15 bp or nucleotides.

2. Nucleic acid fragment according to Claim 1, characterized in that it is adjacent to the CpG sites or in that it contains the CpG sites (EagI, BssHll, Sacll), situated in the 20 kb region surrounding the 125 kb position defined situated at about 100 kb of probe Do33 in the direction of the centromere.

3. Nucleic acid fragment, characterized in that it hybridizes selectively with a nucleic acid fragment according to any one of the Claims 1 or 2, and in that it is DNA or RNA.

4. Nucleic acid fragment according to Claim 1 or Claim 2, characterized in that it hybridizes selectively with an mRNA present in human cells, for example leukocytes, and in that it corresponds to a gene, the 5' end of which is contained in or adjacent to the CpG island at position 125 kb situated at about 100 kb of probe Do33 in the direction of the centromere.

5. Nucleic acid fragment, characterized in that it is the cDNA corresponding to the DNA according to Claim 4.

6. Nucleic acid fragment according to claim 1 or 2 characterized in that it replies to one of the sequences presented in figure 8.

7. Nucleotide probe to detect in vitro mutations responsible for the fragile X syndrome, or other abnormalities resulting from the presence of the said mutations or to localize the gene(s) implicated in the fragile X syndrome, characterized in that it is capable of hybridizing selectively with a nucleic acid fragment according to any one of the Claims 1 to 6 or in that it is capable of hybridizing selectively with such fragment.

8. Nucleotide probe, characterized in that it is selected from among:
- the 9B12 fragments (Eagl-Bgll) of about 7000 bp, 9B12.3 (Pstl-Pstl) of about 1200 bp (Pstl-Pstl) of about 1000 bp, 9B12.5 (Eagl-Pstl) of about 900 bp, 9B12.2 (Pstl-Pstl) of about 1800 bp, stA22 (Avall-Avall) of about 1500 bp, stX21 of about 3800 bp and stX21E (centromeric from the Eagl site);
- the fragments hybridizing selectively with one of the above fragments.

9. Nucleotide probe characterized in that it corresponds to the 9B12.3 fragment comprised between the nucleotides 1 and 1078 of the sequence shown in Figure 8, or any part of this fragment which hybridizes selectively with a nucleic acid fragment according to any one of the Claims 1 to 6.

10. Nucleotide probe characterized in that it corresponds to the 9B12.5 fragment comprised between the nucleotides 1079 and 1985 of the sequence shown in Figure 8, or any part of this fragment which hybridizes selectively with a nucleic acid fragment according to any one of the Claims 1 to 4.

11. Nucleotide probe according to any one of the Claims 8 to 10, characterized in that it is labelled radioactively or by a non-radioactive label, in particular an enzymatic label, a ligand, a fluorescent or luminescent label.

12. mRNA fragment, in particular derived from human leukocytes, characterized in that it hybridizes with a probe according to any one of the Claims 7 to 11.

13. Nucleic acid fragment, characterized in that it hybridizes selectively with a mRNA according to Claim 12.

14. In vitro detection procedure for abnormalities of the DNA resulting from the presence of the mutations associated with the fragile X syndrome or existing in persons not exhibiting clinical symptoms or other abnormalities in the DNA resulting from the presence of these mutations, characterized in that it comprises the following steps :
- the treatment of a sample of genomic DNA with a least one restriction enzyme with a site sensitive to methylation;
- the separation of the fragments by electrophoresis and their hybridization with at least one probe according to any one of the claims 8 to 11 ;
- the visualization of the hybrids formed and the comparison with the results obtained under the same conditions on control fragments.

15. Procedure for the in vitro detection according to Claim 14, characterized in that it further comprises the treatment of the sample of genomic DNA further comprises using of a restriction enzyme with a site insensitive to methylation and in that the visualization step of the hybrids formed and the comparison step lead to the detection of fragments longer than the length of the reference fragments obtained by treatment under the same conditions, starting from a biological sample taken from a normal subject, a non-carrier of mutation at the fragile X site.

16. In vitro diagnostic procedure for the fragile X syndrome or the presence of mutations in carriers without clinical manifestations of mental retardation according to Claim 15, characterized in that the digestion of the genomic DNA is performed with a EagI restriction enzyme with a site sensitive to methylation and a restriction enzyme with a site insensitive to methylation chosen among EcoRI or Hindlll.

17. Process for the in vitro detection of mutations related to the X fragile syndrome or existing in carriers without clinical manifestation or other abnormalities of the DNA resulting from the presence of these mutations, characterized in that it comprises the following steps:
- the treatment of a genomic DNA sample with at least one restriction enzyme with a site insensitive to methylation such as EcoRI or Hindlll;
- the separation of the fragments by electrophoresis and their hybridization with at least one probe according to any one of Claims 8 to 11 ;
- the visualization of the hybrids formed and the detection of fragments longer than the length of the reference fragments obtained by treatment under the same conditions, starting from a biological sample taken from a normal subject, a non-carrier of mutation at the fragile X site.

18. Genetic amplification procedure for a nucleic acid fragment containing mutations or other abnormalities relating to the fragile X site, if necessary resulting from these mutations, characterized in that it consists of binding to the genomic DNA by hybridization two labelled oligonucleotides flanking the said fragment, selected from the nucleic acid fragments of 20 to 30 nucleotides which hybridize selectively with the nucleic acid fragments according to Claim 1 or 2, then of initiating an enzymatic lengthening process with the aid of DNA polymerase, followed by a denaturation process, and of repeating the hybridization - lengthening - denaturation cycle a sufficient number of times to increase the amount of the fragment in an exponential proportion with respect to the number of cycles performed under conditions which allow the visualization of the amplified fragments.

19. In vitro diagnostic procedure for the fragile X syndrome, characterized in that a genetic amplification procedure according to Claim 15 is carried out in parallel on the one hand on the DNA subjected to digestion by an enzyme with a site sensitive to methylation and, on the other, on the DNA not subjected to digestion by this enzyme, then in that the fragments obtained and visualised with ethidium bromide or detected by means of labelling of the oligonucleotide primers are compared.

20. Procedure for the detection of mutation associated with the fragile X syndrome, characterized in that a genetic amplification is carried out according to Claim 18 or 19 of one or more nucleic acid fragments of the 20 kb region surrounding the 125 kb position, in particular of the region containing the sites close to the CpG island, and in that the disappearance or the modification of some of these fragments is detected, in particular in that fragments are detected which are elongated with respect to the size of the fragments obtained under the same conditions of treatment starting from a normal sample, not carrying the fragile X mutation.

21. Recombinant DNA containing at least one nucleic acid fragment according to any one of the Claims 1 to 6, where necessary associated with a sequence making possible the transcription and/or translation in a selected cell host in which the said recombinant DNA is likely to be introduced.

22. Cloning an expression vector characterized in that it contains a recombinant DNA according to Claim 21.

23. Cell host characterized in that it is transformed by a recombinant vector according to Claim 22.

24. Protein resulting from the transcription and translation of a cDNA fragment according to Claim 5 or 6, such as that obtained by the steps:
- transformation of a determined cell host by this cDNA under conditions allowing its transcription and translation;
- recovery of the protein expressed, if necessary after lysis of the cell host.

25. Antibodies directed against a protein according to Claim 21.

26. Kit for the in vitro diagnosis of the X fragile syndrome or of the presence of mutations in carriers without clinical manifestations of mental retardation, comprising:
- oligonucleotides replying to a sequence of a nucleic acid fragment according to any one of Claims 1 to 6, labelled or not, for the amplification of nucleic acid fragments presenting an abnormality resulting from a mutation, in particular a longer DNA and/or nucleic acid fragment containing a site sensitive to methylation according to the invention;
- oligonucleotides labelled or not for the amplification of reference nucleic acid segments;
- linker oligonucleotides;
- buffers, nucleotides and enzymes appropriate for amplification.

## Patentansprüche

1. Nucleinsäurefragment, lokalisiert in der Region Xq27-q28 des X-Chromosoms, dadurch gekennzeichnet, daß es von dem DNA-Segment mit ungefähr 225 kb, definiert zwischen den Sonden St677 (oder DXS463) und Do33 (oder DXS465), umfaßt wird, und dadurch, daß es mindestens 15pb oder Nucleotide enthält.

2. Nucleinsäurefragment nach Anspruch 1, dadurch gekennzeichnet, daß es zu CpG-Positionen benachbart ist oder dadurch, daß es diese CpG-Positionen (EagI, BssHII, SacII) enthält, die sich in der Region von 20 kb um die Position 125 kb befinden, die sich ungefähr 100 kb von der Sonde Do33 in Richtung des Centromers befindet.

3. Nucleinsäurefragment, dadurch gekennzeichnet, daß es selektiv mit einem Nucleinsäurefragment nach einem der Ansprüche 1 oder 2 hybridisiert, und dadurch, daß es sich um DNA oder RNA handelt.

4. Nucleinsäurefragment nach Anspruch 1 oder 2, dadurch gekennzeichnet, daß es selektiv mit mRNA hybridisiert, die in menschlichen Zellen vorhanden ist, z.B. Leukocyten, und dadurch, daß sie einem Gen entspricht, dessen äußerstes 5'-Ende in einer CpG-Insel in Position 125 kb, die sich ungefähr 100 kb von der Sonde Do33 in Richtung des Centromers befindet, enthalten oder einer solchen benachbart ist.

5. Nucleinsäurefragment, dadurch gekennzeichnet, daß es sich um die cDNA handelt, die der DNA gemäß Anspruch 4 entspricht.

6. Nucleinsäurefragment nach Anspruch 1 oder 2, dadurch gekennzeichnet, daß es einer der in Figur 8 dargestellten Sequenzen entspricht.

7. Nucleotidsonde zur in vitro-Bestimmung von Mutationen, die für das X-Mangel-Syndrom verantwortlich sind, oder von anderen Anomalien als Folge der Gegenwart dieser Mutationen, oder zur Lokalisierung des oder der Gene, die mit dem X-Mangel-Syndrom verbunden sind, dadurch gekennzeichnet, daß sie einem Nucleinsäurefragment nach einem der Ansprüche 1 bis 6 entspricht, oder dadurch, daß sie dazu fähig ist, selektiv mit einem solchen Fragment zu hybridisieren.

8. Nucleotidsonde, dadurch gekennzeichnet, daß sie ausgewählt ist unter:
- den Fragmenten 9B12 (EagI-BglI) mit ungefähr 7000bp, 9B12.3 (PstI-PstI) mit ungefähr 1200bp, (PstI-PstI) mit ungefähr 1000bp, 9B12.5 (EagI-PstI) mit ungefähr 900bp, 9B12.2 (PstI-PstI) mit ungefähr 1800bp, stA22 (AvaII-AvaII) mit ungefähr 1500bp, stX21 mit ungefähr 3800bp und stX21E (centromer zur Position EagI), dargestellt in Figur 2B;
- den Fragmenten, die selektiv mit einem der obigen Fragmente hybridisieren.

9. Nucleotidsonde, dadurch gekennzeichnet, daß sie dem Fragment 9B12.3 zwischen den Nucleotiden 1 und 1078 der in Figur 8 dargestellten Sequenz entspricht, oder dem gesamten Teil des Fragments, das selektiv mit einem Nucleinsäurefragment gemäß einem der Ansprüche 1 bis 6 hybridisiert.

10. Nucleotidsonde, dadurch gekennzeichnet, daß sie dem Fragment 9B12.5 zwischen den Nucleotiden 1079 und 1985 der in Figur 8 dargestellten Sequenz entspricht, oder dem gesamten Teil des Fragments, das selekiv mit einem Nucleinsäurefragment nach einem der Ansprüche 1 bis 4 hybridisiert.

11. Nucleotidsonde nach einem der Ansprüche 8 bis 10, dadurch gekennzeichnet, daß sie radioaktiv markiert ist, oder durch einen nicht-radioaktiven Marker, insbesondere einen Enzym-Marker, einen Liganden, einen Lumineszenz- oder Fluoreszenz-Marker, markiert ist.

12. Messenger-RNA, insbesondere aus menschlichen Leukocyten, dadurch gekennzeichnet, daß sie mit einer Sonde gemäß einem der Ansprüche 7 bis 11 hybridisiert.

13. Nucleinsäurefragment, dadurch gekennzeichnet, daß es selektiv mit einer Messenger-RNA nach Anspruch 12 hybridisiert.

14. Verfahren zur in vitro-Bestimmung von DNA-Anomalien als Folge der Gegenwart von Mutationen, die mit dem X-Mangel-Syndrom verbunden sind oder die bei Personen vorhanden sind, die keine klinischen Anzeichen oder andere DNA-Anomalien als Folge der Gegenwart dieser Mutationen zeigen, dadurch gekennzeichnet, daß es die folgenden Stufen umfaßt:
- die Behandlung der genomischen DNA mit mindestens einem Restriktionsenzym für eine gegenüber Methylierung empfindliche Position;
- die Trennung der Fragmente durch Elektrophorese und ihre Hybridisierung mit mindestens einer Sonde nach einem der Ansprüche 8 bis 11;
- die Sichtbarmachung der gebildeten Hybride und Vergleich mit den Ergebnissen, die unter gleichen Bedingungen für Kontrollfragmente erhalten wurden.

15. Verfahren zur in vitro-Bestimmung nach Anspruch 14, dadurch gekennzeichnet, daß die Behandlung der Probe der genomischen DNA außerdem die Verwendung eines Restriktionsenzyms für eine gegenüber Methylierung unempfindlichen Position umfaßt, und dadurch, daß man die Stufe der Sichtbarmachung der gebildeten Hybride und den Vergleich durch Bestimmung von Fragmenten durchführt, die gegenüber den nach den gleichen Bedingungen erhaltenen Kontrollfragmenten länger sind, ausgehend von einer biologischen Probe, die einer normalen Person entnommen wurde, die keine Mutation an der X-Mangel-Position aufweist.

16. Verfahren zur in vitro-Diagnose des X-Mangel-Syndroms oder der Gegenwart von Mutationen bei Personen, die keine klinischen Anzeichen von Geistesschwäche aufweisen, nach Anspruch 15, dadurch gekennzeichnet, daß die Digestion der genomischen DNA durch das Restriktionsenzym EagI für eine gegenüber Methylierung empfindliche Position und ein Restriktionsenzym für eine gegenüber Methylierung unempfindliche Position, ausgewählt unter EcoRI oder HindIII, durchgeführt wird.

17. Verfahren zur in vitro-Bestimmung von Mutationen, die mit dem X-Mangel-Syndrom verbunden sind, oder bei Personen vorhanden sind, die keine klinischen Anzeichen oder andere DNA-Anomalien als Folge der Gegenwart solcher Mutationen zeigen, dadurch gekennzeichnet, daß es die folgenden Stufen umfaßt:
- die Behandlung einer Probe einer genomischen DNA mit mindestens einem Restriktionsenzym für gegenüber Methylierung unempfindliche Positionen, wie EcoRI oder HindIII;
- die Trennung der Fragmente durch Elektrophorese und ihre Hybridisierung mit mindestens einer Sonde nach einem der Ansprüche 8 bis 11;
- die Sichtbarmachung der gebildeten Hybride und die Bestimmung der Fragmente, die länger sind als durch Behandlung unter den gleichen Bedingungen erhaltene Kontrollfragmente, ausgehend von einer biologischen Probe, die einer normalen Person entnommen wurde, die keine Mutation an der X-Mangel-Position aufweist.

18. Verfahren zur genetischen Amplifikation eines Nucleinsäurefragmentes, das Mutationen oder andere Anomalien enthält, in einer Person mit X-Mangel-Position, gegebenenfalls als Folge dieser Mutationen, dadurch gekennzeichnet, daß es darin besteht, daß man an der genomischen DNA durch Hybridisierung zwei markierte Oligonucleotide fixiert, die dieses Fragment flankieren, ausgewählt unter den Nucleinsäurefragmenten mit 20 bis 30 Nucleotiden, die selektiv mit den Nucleinsäurefragmenten nach Anspruch 1 oder 2 hybrisieren, dann eine Enzym-Kettenverlängerungsreaktion mit Hilfe von DNA-Polymerase durchführt, und schließlich eine Denaturierung, und anschließend den Hybridisierungs-Kettenverlängerungs-Denaturierungs-Zyklus so oft wiederholt, daß die Zahl der Zyklen ausreicht, um die Menge des Fragments in einem exponentiellen Verhältnis, bezogen auf die Zahl der durchgeführten Zyklen, zu erhöhen, unter Bedingungen, die die Sichtbarmachung des amplifizierten Fragments ermöglichen.

19. Verfahren zur in vitro-Diagnose des X-Mangel-Syndroms, dadurch gekennzeichnet, daß man parallel ein genetisches Amplifikationsverfahren nach Anspruch 18 durchführt, einerseits mit DNA, die einer Digestion mit einem Enzym für eine gegenüber Methylierung empfindlichen Position unterworfen wurde, und andererseits mit DNA, die keiner Digestion mit diesem Enzym unterworfen wurde, und dadurch, daß man die mit Ethidiumbromid sichtbar gemachten oder mittels einer Markierung der Ausgangsoligonucleotide bestimmten erhaltenen Fragmente vergleicht.

20. Verfahren zur Bestimmung einer mit dem X-Mangel-Syndrom verbundenen Mutation, dadurch gekennzeichnet, daß man eine genetische Amplifikation nach Anspruch 18 oder 19 mit einem oder mehreren Nucleinsäurefragmenten der Region von 20 kb um die Position 125 kb, und insbesondere der Region, die die der CpG-Insel benachbarten Positionen enthält, durchführt, und dadurch, daß man das Verschwinden oder die Modifizierung von bestimmten dieser Fragmente bestimmt, und insbesondere dadurch, daß man die in Bezug auf die Größe der Fragmente verlängerten Fragmente bestimmt, erhalten unter den gleichen Behandlungsbedingungen, ausgehend von einer normalen Probe, die keine X-Mangel-Mutation aufweist.

21. Rekombinante DNA, die mindestens ein Nucleinsäurefragment nach einem der Ansprüche 1 bis 6 enthält, gegebenenfalls mit einer Sequenz assoziert, die die Transkription und/oder Translation in einer Wirtszelle ermöglicht, ausgewählt aus solchen, die zur Einführung dieser rekombinanten DNA geeignet sind.

22. Klonierungs- und Expressions-Vektor, dadurch gekennzeichnet, daß er eine rekombinante DNA nach Anspruch 21 enthält.

23. Wirtszelle, dadurch gekennzeichnet, daß sie durch einen rekombinanten Vektor nach Anspruch 22 transformiert ist.

24. Protein aus der Transkription und der Translation eines cDNA-Fragments nach Anspruch 5 oder 6, erhalten durch die Stufen:
- Transformation einer Wirtszelle durch diese cDNA unter Bedingungen, die ihre Transkription in mRNA und ihre Translation ermöglichen;
- Gewinnung des exprimierten Proteins, gegebenenfalls nach Lyse der Wirtszelle.

25. Antikörper gegen ein Protein nach Anspruch 24.

26. Reagentiensatz zur in vitro-Diagnose von X-Mangel-Syndrom oder dem Vorhandensein von Mutationen bei Personen, die keine klinischen Anzeichen von Geistesschwäche aufweisen, umfassend:
- Oligonucleotide, die einer Sequenz eines Nucleinsäurefragmentes nach einem der Ansprüche 1 bis 6 entsprechen, markiert oder nicht, zur Amplifikation von Nucleinsäurefragmenten, die eine aus einer Mutation resultierende Anomalie darstellen, insbesondere durch erfindungsgemäße Verlängerung von DNA und/oder von Nucleinsäurefragmenten, die eine gegenüber Methylierung empfindliche Position enthalten;
- Oligonucleotide, markiert oder nicht, zur Amplifikation von Nucleinsäuresegmenten als Kontrolle;
- Oligonucleotide, die als "Linker" bezeichnet werden;
- Puffer, Nucleotide und Enzyme für die Amplifikation.
